# EUROPEAN PATENT SPECIFICATION

(11) **EP 2 725 979 B1**
(45) Date of publication and mention of the grant of the patent: **29.05.2019**
(21) Application number: 12805324.6
(22) Date of filing: 15.06.2012
(51) Int. Cl.: G02B 27/09, A61B 5/00, A61B 5/024, A61B 5/0295, G01N 21/31, G02B 5/02

(54) **HOMOGENIZING LIGHT SOURCES IN PHOTOPLETHYSMOGRAPHY**
HOMOGENISIERUNG VON LICHTQUELLEN IN DER PHOTOPLETHYSMOGRAPHIE
HOMOGÉNÉISATION DE SOURCES LUMINEUSES EN PLÉTHYSMOGRAPHIE OPTIQUE

(30) Priority: 29.06.2011 US 201113172569
(43) Date of publication of application: 07.05.2014
(73) Proprietor: Kestrel Labs, Inc., Boulder, Colorado 80301 (US)
(72) Inventor: POLOGE, Jonas, Boulder, Colorado 80301 (US)
(74) Representative: Fairbairn, Angus Chisholm
(86) International application number: PCT/US2012/042778
(87) International publication number: WO 2013/003077

(56) References cited:
- EP-A1- 2 060 227
- EP-A2- 1 080 683
- WO-A1-2010/125705
- US-A- 6 123 719
- US-A1- 2003 007 147
- US-A1- 2004 141 308
- US-A1- 2004 167 499
- US-A1- 2005 228 253
- US-A1- 2011 034 973
- US-A1- 2011 034 973
- US-B1- 6 865 408

## Description

### BACKGROUND-PRIOR ART

**U.S. Patents**

| **Patent Number** | **Kind Code** | **Issue Date** | **Patentee** |
|---|---|---|---|
| 4,407,290 | | Oct 4, 1983 | Wilber |
| 6,115,621 | | Sep 5, 2000 | Chin |
| 6,594,513 | B1 | Jul 15, 2003 | Jobsis |

**U.S. Patent Application Publications**

| **Application Number** | **Kind Code** | **Publication Date** | **Applicant** |
|---|---|---|---|
| 2005/0228253 | A1 | Oct 13,2005 | Debreczeny |
| 2008/0242958 | A1 | Oct 2,2008 | Al-Ali |
| 2010/0113902 | A1 | May 6,2010 | Hete |

### BACKGROUND OF THE INVENTION

In the science of photoplethysmography, light is used to illuminate or trans-illuminate living tissue for the purpose of providing noninvasive measurements of blood analytes or other hemodynamic parameters or tissue properties. In this monitoring modality light is directed into living tissue and a portion of the light which is not absorbed by the tissues, or scattered in some other direction, is detected a short distance from the point at which the light entered the tissue. The detected light is converted into electronic signals that are indicative of the received light intensity exiting the tissue. These signals, one for each emitter or spectral band of light incident on the living tissue ("living tissue" being monitored by photoplethysmography is referred to in this specification as the tissue-under-test), vary with the pulsation of the blood through the tissue-under-test. These time varying signals are referred to as photoplethysmographic signals. The photoplethysmographic signals are used to calculate blood analyte levels such as arterial blood oxygen saturation and/or hemodynamic variables such as heart rate, cardiac output, or tissue perfusion. Among the blood analytes that may be measured by photoplethysmography are various types of hemoglobin, including the percentages of oxyhemoglobin, carboxyhemoglobin, methemoglobin, and reduced hemoglobin in the arterial blood. A device which detects and processes photoplethysmographic signals to measure the levels of various blood analytes and/or various hemodynamic parameters is referred to as a photoplethysmographic measurement apparatus, photoplethysmographic device, or photoplethysmographic instrument.

The first widespread commercially-used photoplethysmographic device in medicine was the pulse oximeter, a photoplethysmographic device designed to measure arterial blood oxygen saturation. In conventional pulse oximetry two different bands of light are used, with each light band possessing a unique spectral content. In early pulse oximetry a tungsten lamp was used to generate broadband light and interference filters were used to isolate two narrow spectral bands of light. Later pulse oximeters replaced the tungsten lamp source and interference filters with two light emitting diodes (LEDs) to generate the desired spectral bands. More recently photoplethysmographic instruments have been developed in which more than two light bands are utilized to allow the measurement of a larger number of blood analytes, including such blood analytes as oxyhemoglobin, carboxyhemoglobin, methemoglobin, and reduced hemoglobin.

For accurate photoplethysmographic measurement of blood analytes it is crucial that the light from all emitters enters the tissue-under-test through the same small aperture. Ideally the light from each emitter is also evenly distributed throughout the aperture. This homogeneous distribution of the light, regardless of the spectral band, emitted through a small aperture, in combination with a small detector aperture, ensures that the light from each emitter is traversing essentially the same sample of tissue-under-test. Conversely, if the light from different emitters can travel different paths through the tissue-under-test then the received pulsatile signal amplitude related to each individual emitter may vary due to the differences in the microvasculature through which the light travels. Any lack of commonality in the optical path from one emitter to another translates directly into measurement error.

The problem of generating a homogeneous distribution of light that is emitted through a small aperture into the tissue-under-test has existed since the earliest days of LED-based pulse oximetry. Given the physical separation of the LEDs in a typical pulse oximetry sensor, the light from each of the emitters could, on average, take a slightly different path though the tissue-under-test. In an attempt to minimize this effect as much as possible, all manufacturers of pulse oximeters positioned the LEDs in the sensors as closely together as possible. Often a diffuser was also added, typically placed some distance from the LEDs, in an attempt to further homogenize the light from the two sources.

The use of a diffuser was disclosed in US patent 4,407,290 where the specification states that "... the light emitted from LEDs 39 and 40 being preferably directed through a light diffusing disc 42 to the blood containing sample 45 to be tested, which sample may be, for example, tissue such as an ear lobe or the like." This use of a diffuser in the sensor design was implemented in the early commercial Ohmeda Biox ear and finger sensors.

The use of a diffuser to homogenize and properly distribute the light over the output aperture was also expressed in US patent 6,115,621 wherein the specification explains that "One type of oximeter sensor will add a diffusing optic to diffuse the light emitted from the light-emitting diodes (LEDs)..." and then proceeds to refer back to the use of the diffuser shown in US patent 4,407,290. The specification for US patent 6,115,621 goes on to state "Also shown is an optional optical diffuser 80 for diffusing the light from emitter 76, which causes a further spreading or mixing of light and may enhance the amount of tissue penetrated in some instances."

A non-photoplethysmographic technology that uses multiple spectral bands of light to measure tissue oxygen saturation is revealed in US patent 6,594,513. This monitoring modality also recognized the need for homogeneous light entering the tissue-under-test and used a "mixer" which "mixes the light from multiple lasers with different wavelengths in order to enter it homogeneously into the scalp from a single aperture 18." This particular diffuser used a "clear material, such as a stiff silicone gel, containing white, (i.e. non-absorbing and wavelength independent) scattering particles, such as titanium dioxide powder, which disperses the light entering the mixer."

Similarly, US patent application publication number 2008/0242958 recognizes the need for proper diffusion of the light incident on the tissue-under-test. This application states that "In various embodiments a diffuser scatters the radiated light so that a tissue site is uniformly illuminated across all of the wavelengths."

An MRI compatible photoplethysmographic sensor is disclosed in the US patent application publication number US 2010/0113902. In this sensor the light sources, or emitters, are optical fibers which deliver the light from LEDs to the sensor. The applicant states that the sensor may include "...a diffuser between the fiber optic material and the animal tissue."

In these five references the diffuser is substantially positioned against the skin some distance from the LEDs or light sources (or, in one case, filling the space from the light sources to the output aperture). This configuration has a problem in that it does not fully homogenize the light across the output aperture. (The output aperture, or exit aperture, being the surface area through which the light is transmitted from the sensor into the tissue-under-test. The aperture is also intended to block light from exiting from any other point on the sensor and entering the tissue-under-test.) Mapping the intensity of the individual light sources as a function of position on the output side of the aperture would show a non-uniform intensity profile across the output aperture and, worse yet from the standpoint of measurement accuracy, the intensity mapping would vary from one light source to another. Ideally fully homogenized light, intended for illumination of the tissue-under-test, would not exhibit any variation in output intensity as a function of position of measurement on the output surface of the output aperture, regardless of which light source was being measured.

A second problem with the method of mixing the light defined in these prior art references, and seen in the early Ohmeda Biox sensors, is in the actual design of the diffusing element. To the extent that the diffusers are made up of scattering (or light reflecting) elements randomly suspended in a clear transmitting medium, the light transmission losses are relatively large. Furthermore, the more effective this type of diffuser is at mixing the incident light, the worse the optical throughput. This loss in light intensity out of the sensor has several detrimental effects. It means either degradation in signal-to-noise level or that the light sources must emit more light to ensure sufficient optical signal strength after the light passes through the tissue-under-test. This typically means more input power to drive the LEDs or other light generating elements such as lasers or tungsten lamps. Higher input power results in shorter lifetimes of the light generating elements, more heat generation, and even shorter operating times for battery-powered devices.

US patent application publication number 2005/0228253 reveals a different method of mixing the light where a set of input fiber bundles carries light from each of a number of individual light generating elements to an N-to-1 fiber coupler where all the input fibers are coupled into a single larger output fiber. At the N-to-1 fiber coupler the individual fibers, originating from any given light generating element, are spatially distributed evenly throughout the N-fiber bundle thereby, to some extent, homogenizing the light into the single output fiber. This solution suffers from the problem of incomplete homogenization of the light as the mixing is limited by the discrete nature and size of the input fibers. Furthermore this solution is high in cost, requires a great deal of physical space, and, potentially, a great many optical fibers. And, finally, the large diameter of the single output fiber in the coupler creates a cable that is inflexible and prone to breakage during clinical use.

Furthermore, patent applications US 2011/034973(A1), EP 2060227 (A1) and US patent 6865408 (B1) disclose similar optical diagnostic devices.

Whereas conventional pulse oximetry sensors have had the problem of mixing only two light sources together, more advanced photoplethysmographic devices are now using many more emitters to measure ever more blood analytes. In addition, some of these analytes have lower optical absorption thus requiring more sensitive and accurate photoplethysmographic instruments to make measurements with clinically-meaningful resolution, precision, and accuracy. A crucial element in the design of an accurate photoplethysmographic measurement apparatus is a light homogenizing apparatus that puts out well homogenized light, from multiple emitters, at a low cost, and with relatively high optical efficiency (high optical efficiency being a low loss from optical power-in to optical power-out). An additional design requirement of the light homogenizing apparatus may also be that it is physically small in size to allow it to be integral to the sensor, or sensor cable, where it could be positioned at, on, or near, the tissue-under-test.

### BRIEF SUMMARY OF THE INVENTION

In accordance with one embodiment a light homogenizing apparatus for a photoplethysmographic device comprises one or more light guides conducting light from at least one laser wherein the light exiting the light guides is incident on a light mixing element. The light mixing element comprises a plurality of one or more light sources incident upon a diffusing element followed by an internally reflective light guide. Accordingly, several advantages of one or more aspects are as follows: that light exiting the light mixing element is a substantially homogeneous mixture of the light that was incident on the light homogenizing apparatus; and that the apparatus provides a high optical throughput. The combination of these advantages contributes to accurate, high-resolution photoplethysmographic measurement.

### DRAWINGS

*FIG. 1a**.* Basic light homogenizing apparatus.
*FIG. 1b**.* Basic light homogenizing apparatus. Side view with representative optical ray trace.
*FIG. 2a**.* Light homogenizing apparatus with folded optical path.
*FIG. 2b**.* Light homogenizing apparatus with folded optical path. Side view with representative optical ray trace.
*FIG. 3a**.* Light homogenizing apparatus with folded optical path and integral diffractive element on prism.
*FIG. 3b**.* Light homogenizing apparatus with folded optical path and integral diffractive element on prism. Side view with representative optical ray trace.

### DETAILED DESCRIPTION OF THE INVENTION

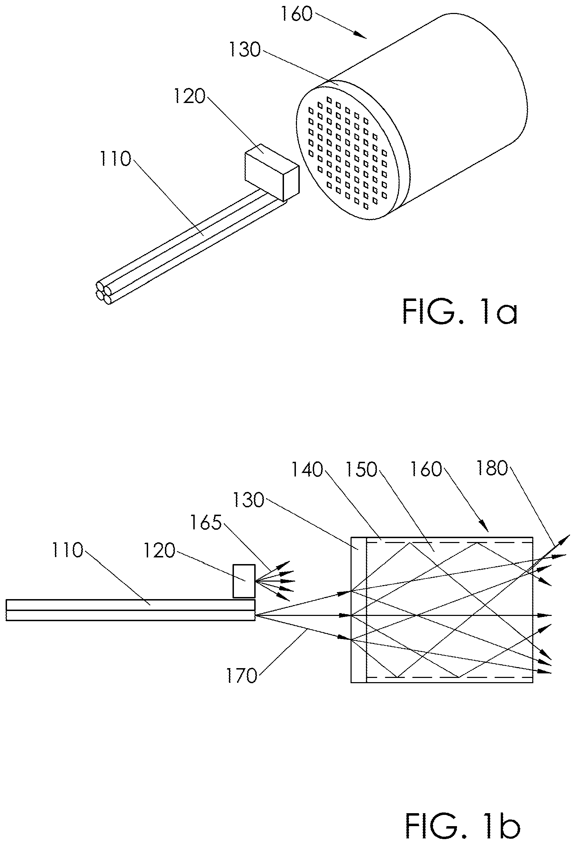

One exemplary construction of a light homogenizing apparatus for a laser-based photoplethysmographic device is shown in FIG. a and FIG. 1b. FIG. 1a is an isometric view of this example and FIG. 1b is a side view of the same example including lines indicating optical rays passing through the apparatus.

In these two figures light is conducted down a set of one or more light guides **110** toward a light mixing element. The light mixing element is made up of the combination of a diffuser **130** and an internally reflective light guide **160.** Alternatively, or in addition to the light guides **110**, light may also be generated by one or more discrete light sources **120.** Light **170** exiting the light guides **110** or light **165** being emitted by the discreet light sources **120** is incident upon the diffuser **130** portion of the light mixing element. The diffuser **130** increases the angular dispersion of light **(165** and **170)** entering the internally reflective light guide **160.** Finally, homogenized light **180**, a well mixed version of the input light **165** and **170**, exits the internally reflective light guide.

The internally reflective light guide **160** is a combination of a light pipe **150** and a reflector **140** that reflects light incident upon it back into the light pipe **150.** The reflector **140** substantially surrounds all surfaces of the light pipe **150** with the exception of the input and the output surfaces as shown in the side view of the apparatus in FIG. 1b.

It is this homogenized light **180** exiting the internally reflective light guide **160** portion of the light mixing element that would then be made incident upon a tissue-under-test for use in photoplethysmographic measurement. Ideally output light **180** is a completely homogeneous mixture of light from all of the input emitters, regardless of whether those emitters are the one or more light guides **110** or the one or more discrete light sources **120** or any combination of the two.

A homogeneous mixture refers to a distribution of light intensity such that, at any point on the output surface (or the output side of the output aperture) of the light mixing element **160,** the light does not vary in intensity as a function of position on that output surface. Ideally a high degree of homogeneity would be maintained whether any one individual emitter is turned on or any combination of emitters is turned on.

Functionally, at least one of the light sources is a laser source. In addition to a laser source, other types of light sources can be used concurrently in the apparatus. These could include, for example, light emitting diodes, tungsten or other filament type lamps, or gas lamps. The laser source could be a semiconductor laser, a gas laser, or nearly any other type of laser where the laser light can be directed or launched into a light guide **110** or where the laser can act as a discrete light source **120.** The light guide **110** can be any of a number of different elements that conduct light from one point to another including glass or plastic optical fibers, a liquid light guides, plastic light pipes, or other such elements.

Light emitted from the light guides **110** or from the discrete light sources **120** is then incident upon a diffuser **130.** While FIG. 1a and 1b do not show a mechanical mounting system for the components shown in these figures, they are mechanically held in a position to optimize several different properties simultaneously. Two of the more important properties to optimize in this apparatus are the throughput or optical efficiency of the system and the homogeneity of the output light. By separating the light emitters **110** and **120** from the diffuser **130** by a short distance, just as was done in the early pulse oximeter sensors, the light **165** and **170** from these emitters is able to spread out to cover more of the surface area of the diffuser **130.** This helps to begin to homogenize the light traveling through the system. But the light sources **110** and **120** should also be close enough to the diffuser **130** to minimize light lost due to missing the input aperture of the diffuser **130.** The exact distance of the emitters **110** and **120** to the diffuser **130** will therefore be selected based upon the numerical aperture (NA) of the emitters and the diameter or surface area of the input face of the diffuser **130.**

The diffuser **130** then further disperses (i.e. the scatters and spreads in different directions) the light as is indicated by the ray trace shown in FIG. 1b. In this figure three "rays" of light **170** being emitted from light guides **110** are incident upon diffuser **130.** The diffuser **130** then spreads out each ray of light incident upon it. This means that light from every point on the diffuser is directed into an internally reflective light guide **160** over a wide range of angles. The internally reflective light guide **160** is made up of a light pipe **150** and a reflector **140.** The dispersed light exiting the diffuser **130** and entering the light pipe **150** can then reflect off the reflector **140**, further mixing or homogenizing the light. Finally, well homogenized light **180** exits the light pipe and, if incident on living tissue, can be used to make photoplethysmographic measurements.

One way in which the internally reflective light guide **160** can function is to utilize the principle of total internal reflection (TIR). In this case the internally reflective light guide **160** would not require reflector **140.** This TIR method will work efficiently only if light entering the light pipe **150** is at an angle less than the NA of the light pipe **150.** TIR can also be used if the optical power budget of the system can afford the loss of any light entering the internally reflective light guide **160** at an angle greater than the NA of the internally reflective light guide **160.**

If the angle of portions of the light entering the internally reflective light guide **160** is greater than the angle that can be supported by TIR, a reflector **140** can be added to the light pipe **150.** This allows a high dispersion angle of light to be created by the combination of the spatial irradiance pattern of the emitters **110** and **120** and the diffuser **130.** High angle light rays are then reflected by the reflector **140** back into the light pipe **150.** Note that internally reflective light guide **160** can be a hollow cylinder (or other hollow shape), a glass or plastic light guide, or a liquid light guide, forming the light pipe **150** surrounded by the reflector **140.**

The internally reflective behavior of the internally reflective light guide **160** maximizes the homogenizing effect of this apparatus and is shown graphically by the ray trace lines in FIG. 1b. The internal reflective nature of the internally reflective light guide **160** provided by the reflector **140** further allows a high degree of homogeneity of light to be obtained in a very short physical distance, thus allowing the entire light mixing element to be a very small optical element easily mounted on, or designed to be integral to, a photoplethysmographic sensor or cable.

An additional benefit of this apparatus is that it can also simultaneously provide a fixed output aperture. The reflector **140** blocks any light from escaping the light pipe **150** except where it is not surrounded by the reflector **140.** By designing the sensor so that the internally reflective light guide **160** is positioned against the tissue-under-test, the reflector **140** also provides the function of creating a fixed output aperture for homogenized light **180** exiting the light homogenizing apparatus.

To further improve the optical performance of the light homogenizing apparatus one can optimize the design of the diffuser itself. In prior art diffusers used in photoplethysmographic instrumentation, a scattering medium is suspended in an otherwise optically clear substrate material to "diffuse" or scatter the light in all directions. Such diffusers, by their very nature, also scatter light back toward the light sources, thus making them inefficient with low optical throughput. An alternative solution that further enhances the optical throughput of the light homogenizing apparatus is to replace the conventional scattering-based diffuser with a patterned surface diffuser.

Patterned surface diffusers are essentially a diffractive element engineered to diffuse light in a predetermined manner. A patterned surface diffuser disperses light in an engineered manner by providing an engineered pattern or micro structure embedded in one surface of the diffuser. Some of the advantages of a patterned surface diffuser include high optical throughput and controlled dispersion of the input light.

"Controlled dispersion" refers to the ability to select the angular dispersion of light exiting the diffuser. For example, a diffuser can be engineered to cause light incident upon it, at an angle perpendicular to its diffusing surface, to be distributed evenly over a predetermined solid angle. For example, if diffuser **130** in FIG. 1b is a patterned surface diffuser designed to provide a distribution angle of 30 degrees (1/3 π steradian), then light **170** or **165** normally incident upon the patterned surface diffuser **130** is distributed over a solid angle of 1/3 π steradian as it exits the diffuser and enters the light pipe **150.**

Using a patterned surface diffuser, and selecting the length and cross sectional profile of the light pipe **150**, the designer can control the maximum number of reflections off the reflector **140.** In FIG. 1b, for example, with a controlled distribution angle out of the patterned surface diffuser of approximately 30 degrees, only the most marginal rays, entering the light pipe at the highest angle from the normal to the surface of the patterned surface diffuser, will experience more than a single reflection. Because no reflection off of a physical surface can be completely lossless, this minimizes light losses due to multiple imperfect reflections.

An additional advantage to the patterned surface diffuser is that the patterned surface diffuser **130** can be integrated into the light pipe **150**; i.e. the patterned surface diffuser can be constructed on the input end of the light pipe **150.** This integrated design has the potential to be less expensive than individual elements, easier to manufacture, and eliminates any possible air gap between the two elements that would create Fresnel reflections and reduce the optical throughput of the apparatus.

If the diffuser **130** is not integrated into the light pipe **150**, any potential air gap between the diffuser **130** and the light pipe **150** can alternatively be eliminated by using an optically clear adhesive or index-matching material between the two components to minimize changes in index of refraction that cause Fresnel reflections as the light travels through the system.

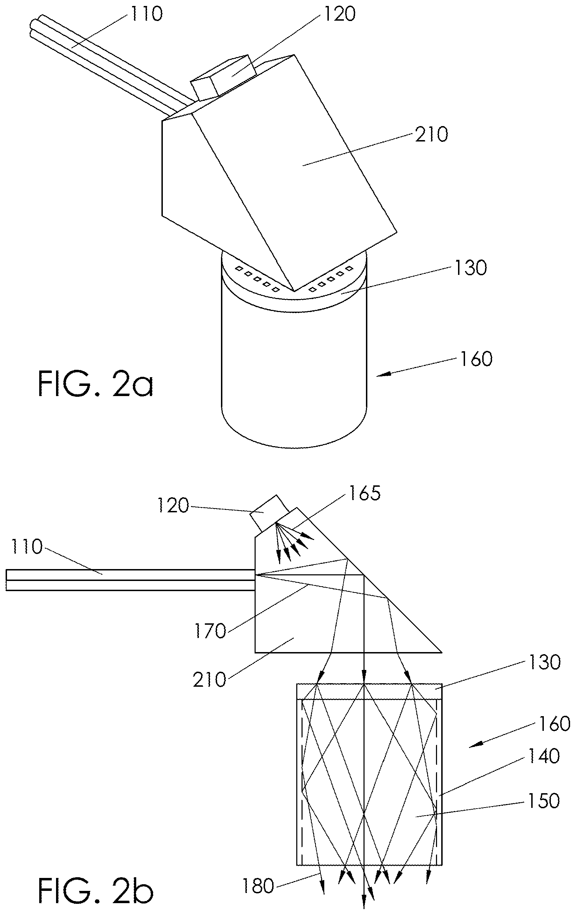

FIG. 2a, an isometric view, and FIG. 2b, a side view showing the optical ray trace, provide an exemplary construction of a light homogenizing apparatus that incorporates a folded optical path. In this exemplary construction light exiting one or more light guides **110**, or being emitted by one or more discrete light sources **120**, is coupled or launched into a prism **210.** Light emitted from at least one of the light guides **110**, or emitted from at least one of the discrete light sources **120**, is laser light.

As shown in FIG. 2b the prism **210** receives light **165** or **170** emitted by emitters **110** or **120** and reflects this light off the 45 degree surface toward the light mixing element. Note that light **165** may enter the prism **210** at a different angle than light **170** and therefore portions of this light may not require reflection off the 45 degree surface to be incident upon the light mixing element.

The 45 degree surface of the prism **210** may be reflective based only on the principle of total internal reflection or it may be coated with a reflector to allow any light incident upon that surface (at any angle with respect to the normal of that 45 degree angle surface) to be internally reflected. It should be noted that the prism **210** could alternatively be replaced with a mirror, or mirrored surface, placed at approximately the same position as the 45 degree angle surface of prism **210** to achieve the same optical behavior.

The prism **210**, as would a mirror, allows light from the two different types of emitters **110** and **120** to be introduced into the system from two different orientations as illustrated in FIG. 2a and FIG. 2b. It also folds the optical path approximately 90 degrees to allow for a more ergonomically comfortable design of the sensor cable in certain circumstances, for example, when it is more comfortable to have the sensor cable run parallel to the surface of the tissue-under-test. This same sensor cable run could be accomplished by curving the light guides **110** over a 90 degree arc, but minimum bend radius limitations of the light guides **110** may create a sensor incorporating the light homogenizing apparatus of this design to be larger and therefore more clumsy.

Other than having a folded optical path, the design shown in FIG. 2a and FIG. 2b is functionally very similar to that shown in FIG. 1a and FIG. 1b. Light **165** or **170** exits the prism **210** and is incident upon diffuser **130.** Diffuser **130** could be a conventional diffuser or a patterned surface diffuser. The diffuser **130** increases the spread, or angular distribution, of light **165** or **170** being launched into light pipe **150.** Light pipe **150** is surrounded by a reflector **140,** which may, for example, be a reflective film, or a reflective coating of a material such as gold or aluminum. Together the light pipe **150** and the reflector **140**, create an internally reflective light guide **160.**

The reflector **140** may also function to provide the aperture for the homogenized output light **180.** A well controlled and relatively small optical aperture for light entering the tissue-under-test is preferred as it increases the accuracy of photoplethysmographic measurements in living tissue.

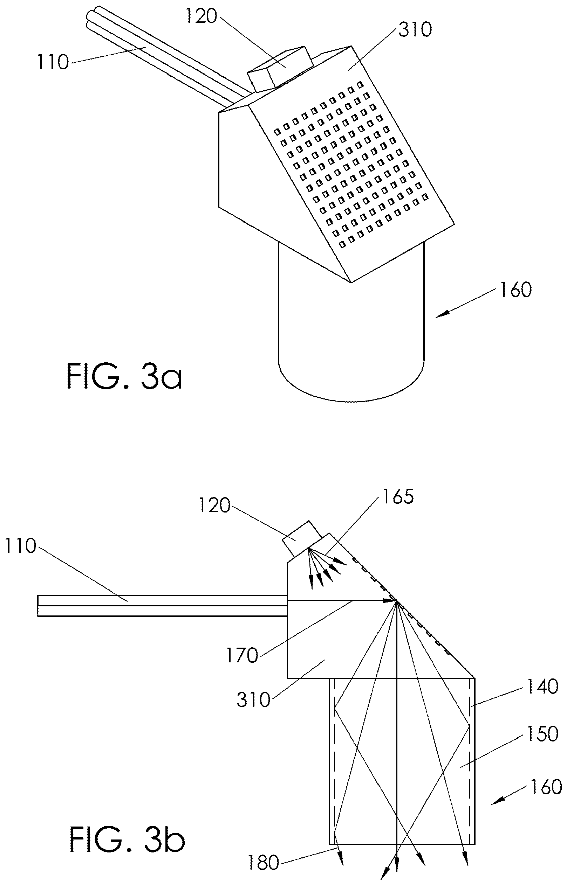

The exemplary construction of the optical design of the light homogenizing apparatus shown in isometric view in FIG. 3a, and as a side view with ray trace in FIG. 3b, has increased optical efficiency over the previous two exemplary construction shown. In this exemplary construction again light **170** exits one or more light guides **110**, and light **165** exits any discrete light sources **120.** Light **170** (and possibly some light **165** from any discrete light sources **120**) is incident on the 45 degree surface of prism **310.** This prism is different from prism **210** in that the 45 degree angle surface of prism **310** is designed to incorporate a patterned surface diffuser. Again, as was the case with prism **210,** the 45 degree angle surface of the prism **310** may be coated with a reflective material. The light reflecting off the 45 degree angle surface of the prism **310** is diffused out (or dispersed) before entering an internally reflective light guide **160** made up of a light pipe **150** surrounded by a reflector **140.**

The increase in throughput, or optical efficiency, of this exemplary construction results from, at a minimum, the elimination of two changes in index of refraction with resulting Fresnel reflection losses that exist in the light paths of the previous two exemplary constructions shown in FIG. 1a and FIG. 1b and FIG. 2a and FIG. 2b. In these earlier exemplary constructions there is an air gap between the emitters **110** or **120** and the diffuser **130.** The exemplary construction shown in FIG. 3a and FIG. 3b does not have this air gap, thereby resulting in a higher efficiency optical path with the same well homogenized light **180** exiting the internally reflective light guide **160.**

Laser-based light sources are now being employed in photoplethysmographic instruments to improve measurement accuracy and to further increase the number of blood analytes that can be effectively and accurately measured by this monitoring modality. But when laser light travels down a light guide, often the distribution of the laser light within the light guide is non-uniform, particularly if the light guide is a multimode optical fiber. This is referred to as a non uniform modal distribution. The use of laser light in photoplethysmography therefore further increases the need to homogenize the light before it is incident on the tissue-under-test.

In past generations of LED-based photoplethysmographic devices a high degree light mixing was not as important. Simply placing the two LED die in very close proximity to one another in the emitter assembly, and providing a single aperture for the output light, was sufficient. In the earliest pulse oximetry sensors, when LEDs were not yet used in die form and pre-packaged LEDs were used in the sensor emitter assembly, a simple diffuser, encircled by an output aperture and placed a short distance from the LEDs, provided sufficient light mixing for the relatively crude measurement of oxygen saturation.

As newer generations of photoplethysmographic devices attempt to make ever more accurate measurements of multiple blood analytes, a more homogeneous mixture of light must be launched into the tissues to ensure that the light from all light sources follows essentially the same optical path through the tissue. The apparatus of this invention supplies this required homogeneity, in part, by following the diffusing element **130** (or the 45 degree diffusing surface of prism **310)** with the internally reflective light guide **160.**

The previous discussion of the embodiments has been presented for the purposes of illustration and description. The description is not intended to limit the invention to the form disclosed herein. Variations and modifications commensurate with the above are considered to be within the scope of the present disclosure. The embodiments described herein are further intended to explain the best modes presently known of practicing the invention and to enable others skilled in the art to utilize the invention as such, or in other embodiments, and with the particular modifications required by their particular application or uses of the invention.

## Claims

1. A photoplethysmographic device comprising a light homogenizing apparatus, the light homogenizing apparatus comprising:
a. one or more light guides (110) conducting light from at least one laser;
b. a light mixing element comprising at least a diffuser (130) followed by an internally reflective light guide (160), the internally reflective light guide (160) comprising a light pipe (150) and a reflector (140) that reflects light incident upon it back into the light pipe (150) and that substantially surrounds all surfaces of the light pipe (150) with the exception of the input and the output surfaces;
wherein light exiting the one or more light guides (110) is incident on the diffuser (130) of the light mixing element, and the light exiting the diffuser (130) enters the internally reflective light guide (160).

2. The device of claim 1, further comprising one or more discrete light sources (120), wherein light exiting the one or more discrete light sources (120) is incident on the diffuser (130) of the light mixing element.

3. The device of claim 2 wherein the discrete light source (120) is a light emitting diode.

4. The device of claim 1 wherein the diffuser (130) is a patterned surface diffuser.

5. The device of claim 1 wherein the internally reflective light guide (160) further provides an output aperture.

6. The device of claim 1 wherein the diffuser (130) is integrated into the internally reflective light guide (160).

7. A method for homogenizing light delivered by a photoplethysmographic measurement system to a tissue-under-test comprising the steps of:
a. providing a plurality of emitters including at least one light guide (110) conducting light from a laser;
b. arranging the plurality of emitters to cause light exiting the plurality of emitters to be incident on a diffuser (130);
c. positioning an internally reflective light guide (160) at the output of the diffuser (130) so that the light exiting the diffuser (130) enters the internally reflective light guide (160), the internally reflective light guide (160) comprising a light pipe (150) and a reflector (140) that reflects light incident upon it back into the light pipe (150) and that substantially surrounds all surfaces of the light pipe (150) with the exception of the input and the output surfaces.

8. The method of claim 7 wherein the step of arranging the plurality of emitters to be incident on a diffuser (130) further comprises the step of arranging the plurality of emitters to be incident on a patterned surface diffuser.

9. The method of claim 7 wherein the step of positioning an internally reflective light guide (160) further comprises configuring the internally reflective light guide (160) to provide an exit aperture.

10. The method of claim 7 wherein the step of positioning an internally reflective light guide (160) further comprises arranging the internally reflective light guide (160) so that no air gap exists between the diffuser (130) and the internally reflective light guide (160).

## Patentansprüche

1. Photoplethysmographische Vorrichtung, umfassend eine Lichthomogenisierungsvorrichtung, wobei die Lichthomogenisierungsvorrichtung umfasst:
a. einen oder mehrere Lichtleiter (110), die Licht von mindestens einem Laser leiten;
b. ein Lichtmischelement mit mindestens einem Diffusor (130), gefolgt von einem innenreflektierenden Lichtleiter (160), wobei der innenreflektierende Lichtleiter (160) einen Hohllichtleiter (150) und einen Reflektor (140) aufweist, der darauf einfallendes Licht zurück in den Hohllichtleiter (150) reflektiert und der im Wesentlichen alle Flächen des Hohllichtleiters (150) mit Ausnahme der Eingangs- und der Ausgangsfläche umgibt;
wobei Licht, das den einen oder die mehreren Lichtleiter (110) verlässt, auf den Diffusor (130) des Lichtmischelements einfällt, und das den Diffusor (130) verlassende Licht in den innenreflektierenden Lichtleiter (160) eintritt.

2. Vorrichtung nach Anspruch 1, ferner umfassend eine oder mehrere diskrete Lichtquellen (120), wobei aus der einen oder den mehreren diskreten Lichtquellen (120) austretendes Licht auf den Diffusor (130) des Lichtmischelements fällt.

3. Vorrichtung nach Anspruch 2, wobei die diskrete Lichtquelle (120) eine Leuchtdiode ist.

4. Vorrichtung nach Anspruch 1, wobei der Diffusor (130) ein strukturierter Oberflächendiffusor ist.

5. Vorrichtung nach Anspruch 1, wobei der innenreflektierende Lichtleiter (160) ferner eine Ausgangsöffnung bereitstellt.

6. Vorrichtung nach Anspruch 1, wobei der Diffusor (130) in den innenreflektierenden Lichtleiter (160) integriert ist.

7. Verfahren zum Homogenisieren von Licht, das von einem photoplethysmographischen Messsystem an ein zu testendes Gewebe abgegeben wird, umfassend die Schritte:
a. Bereitstellen einer Vielzahl von Emittern, die mindestens einen Lichtleiter (110) einschließen, der Licht von einem Laser leitet;
b. Anordnen der Vielzahl von Emittern, um zu bewirken, dass Licht, das die Vielzahl von Emittern verlässt, auf einen Diffusor (130) einfällt;
c. Positionieren eines innenreflektierenden Lichtleiters (160) am Ausgang des Diffusors (130), so dass das aus dem Diffusor (130) austretende Licht in den innenreflektierenden Lichtleiter (160) eintritt, wobei der innenreflektierende Lichtleiter (160) einen Hohllichtleiter (150) und einen Reflektor (140) aufweist, der auf ihn einfallendes Licht in den Hohllichtleiter (150) reflektiert und der alle Oberflächen des Hohllichtleiters (150) mit Ausnahme der Eingangs- und der Ausgangsfläche umgibt.

8. Verfahren nach Anspruch 7, wobei der Schritt des Anordnens der Vielzahl von Emittern, die auf einen Diffusor (130) einfallen sollen, ferner den Schritt des Anordnens der Vielzahl von Emittern umfasst, um auf einen strukturierten Oberflächendiffusor einzufallen.

9. Verfahren nach Anspruch 7, wobei der Schritt des Positionierens eines innenreflektierenden Lichtleiters (160) ferner das Konfigurieren des innenreflektierenden Lichtleiters (160) umfasst, um eine Austrittsöffnung bereitzustellen.

10. Verfahren nach Anspruch 7, wobei der Schritt des Positionierens eines innenreflektierenden Lichtleiters (160) ferner das Anordnen des innenreflektierenden Lichtleiters (160) umfasst, so dass kein Luftspalt zwischen dem Diffusor (130) und dem innenreflektierenden Lichtleiter (160) besteht.

## Revendications

1. Dispositif photopléthysmographique comprenant un appareil d'homogénéisation de la lumière, l'appareil d'homogénéisation de la lumière comprenant:
a. un ou plusieurs guides de lumière (110) conduisant la lumière d'au moins un laser;
b. un élément de mélange de lumière comprenant au moins un diffuseur (130) suivi d'un guide de lumière à réflexion interne (160), le guide de lumière à réflexion interne (160) comprenant un conduit de lumière (150) et un réflecteur (140) réfléchissant la lumière incidente après son retour dans le conduit de lumière (150) et qui entoure sensiblement toutes les surfaces du conduit de lumière (150) à l'exception des surfaces d'entrée et de sortie;
dans lequel la lumière sortant du ou des guides de lumière (110) est incidente sur le diffuseur (130) de l'élément de mélange de lumière, et la lumière sortant du diffuseur (130) pénètre dans le guide de lumière à réflexion interne (160).

2. Dispositif selon la revendication 1, comprenant en outre une ou plusieurs sources de lumière discrètes (120), dans lequel la lumière sortant de la une ou des plusieurs sources de lumière discrètes (120) est incidente sur le diffuseur (130) de l'élément de mélange de lumière.

3. Dispositif selon la revendication 2, dans lequel la source de lumière discrète (120) est une diode électroluminescente.

4. Dispositif selon la revendication 1, dans lequel le diffuseur (130) est un diffuseur à surface configurée.

5. Dispositif selon la revendication 1, dans lequel le guide de lumière à réflexion interne (160) fournit en outre une ouverture de sortie.

6. Dispositif selon la revendication 1, dans lequel le diffuseur (130) est intégré dans le guide de lumière à réflexion interne (160).

7. Procédé d'homogénéisation de la lumière délivrée par un système de mesure photopléthysmographique à un tissu sous test comprenant les étapes consistant à:
a. fournir une pluralité d'émetteurs comprenant au moins un guide de lumière (110) conduisant la lumière provenant d'un laser;
b. agencer la pluralité d'émetteurs pour que la lumière sortant de la pluralité d'émetteurs soit incidente sur un diffuseur (130);
c. positionner un guide de lumière à réflexion interne (160) à la sortie du diffuseur (130), de sorte que la lumière sortant du diffuseur (130) pénètre dans le guide de lumière à réflexion interne (160), le guide de lumière à réflexion interne (160) comprenant un conduit de lumière (150) et un réflecteur (140) qui réfléchit la lumière incidente dans le conduit de lumière (150) et qui entoure sensiblement toutes les surfaces du conduit de lumière (150) à l'exception des surfaces d'entrée et de sortie.

8. Procédé selon la revendication 7, dans lequel l'étape consistant à agencer la pluralité d'émetteurs de manière à être incidente sur un diffuseur (130) comprend en outre l'étape consistant à agencer la pluralité d'émetteurs de manière à être incidente sur un diffuseur à surface structurée.

9. Procédé selon la revendication 7, dans lequel l'étape de positionnement d'un guide de lumière à réflexion interne (160) comprend en outre la configuration du guide de lumière à réflexion interne (160) pour fournir une ouverture de sortie.

10. Procédé selon la revendication 7, dans lequel l'étape de positionnement d'un guide de lumière à réflexion interne (160) comprend en outre l'agencement du guide de lumière à réflexion interne (160), de sorte qu'il n'y ait aucun intervalle d'air existant entre le diffuseur (130) et le guide de lumière à réflexion interne (160).
